# EUROPEAN PATENT APPLICATION

(11) **EP 1 216 619 A2**
(43) Date of publication of application: **26.06.2002**
(21) Application number: 01205061.3
(22) Date of filing: 20.12.2001
(51) Int. Cl.: A23C 19/032, A23C 19/06, C12N 15/74

(54) **Method for the preparation of a cheese product**

(30) Priority: 20.12.2000 EP 00204684
(71) Applicant: Campina B.V., 5300 CC Zaltbommel (NL)
(72) Inventor: Bruinenberg, Paul Gerard, 5071 KE Udenhout (NL)
(74) Representative: Wittop Koning, Tom Hugo

(57) **Abstract**

Described is a method for the preparation of cheese and cheese based products comprising the step of adding cystathionine-β-lyase (CBL) overproducing bacteria, such as recombinant CBL overproducing bacteria, to material from which the cheese product is formed and a cheese product, obtainable by the said method. The cheese product has an enhanced sulphur, sweet, brothy and fruity taste compared to a corresponding conventionally produced cheese product.

## Description

The present invention relates to a method for the preparation of a cheese product, having an improved taste and aromatic properties.

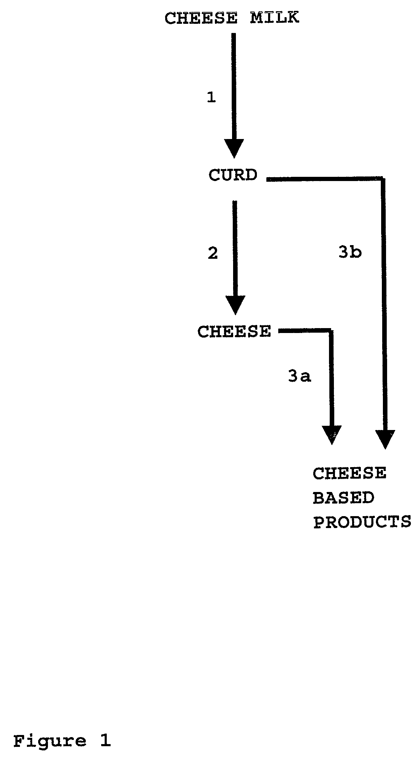

In the art, methods for the preparation of cheese products are generally known, and involve curding milk (the so-called "cheese milk") and ripening of the curd into cheese products using lactic acid bacteria. The main steps in a standard cheese making process are elucidated in figure 1. With "cheese products" products are meant that are obtainable from curd that has been subjected to a ripening process. The term "cheese-products" therefore encompasses e.g. curd cheese, common cheese, such as cottage cheese, Gouda, Parmesan, Cheddar, etc., and varieties thereof, as well as cheese based products, such as processed cheese products, cheese analogues, cheese paste and enzyme modified cheese. The present invention is of particular relevance for the preparation of cheese products of the semi-hard type, such as cheddar and Gouda-type cheese. The above mentioned terms are known in the art; for an overview of the above, see the textbook "Cheese: chemistry, physics and microbiology", 2^{nd} edition, P.F. Fox, ed. Chapman and Hall, London, Great-Britain, 1993.

Lactic acid bacteria, such as Lactococci, Lactobacilli, Leucostonocs and *Streptococcus thermophilus* play an important role in the different process steps of cheese preparation, such as in the curding process. In the process, milk proteins are digested into peptides, that subsequently are cleaved into individual amino acids. These amino acids already confer a basic taste to the cheese. In addition, said amino acids can be converted into more or less volatile compounds, some of which comprising sulphur, that confer the typical cheese taste during the ripening process.

In this respect, the enzyme cystathione-β-lyase (CBL, EC4.4.1.8) is e.g. identified in Lactococcus (Alting et al (1995) Appl. Environ. Microbiol., 61, pp 4037-4042). Said enzyme catalyses the reaction from L-cystationine to homocysteine, as well as the reaction from L-methionine to methanethiol. The methanethiol formation is thought to be responsible for the production of volatile aromatic compounds in many cheese types such as cheddar and Gouda, leading to the typical characteristic cheese taste. CBL is produced by the conventional lactic acid bacteria in relatively low amounts (0,014 U/mg protein; see for the definition of CBL units Alting et al, supra. However, according to Alting, supra, the substrate specificity of CBL for L-cystationine is a 100 fold higher than for L-methionine. Further, the activity of CBL at the pH of cheese preparation conditions *in vitro* (pH of 5.0-5.5) appeared to be only 10-15% of the activity at optimum pH (pH 7.5-8.5).

Fernandez et al (Appl. Environ. Microbiol., 66, pp 42-48 (2000)) have constructed a *Lactococcus lactis* strain, overproducing CBL. Said strain was tested *in vitro* at optimal conditions (pH 7), showing an enhanced CBL activity compared to the wild type parent strain. However, no data exist regarding CBL-activity at cheese preparation conditions. Further, by the engineered Lactococcus strain of Fernandez, CBL was produced in a non-food grade manner.

Further, Diaz et al (Appl. Environ. Microbiol., 64, pp 3320-3326 (1998)) have tested CBL activity at mimicked cheese preparation conditions *in vitro* (pH 5.0-5.2 and an additional NaCl concentration of 3 to 5%). It was found that at such cheese preparation conditions, in contrast to optimum conditions at pH 7.2 without additional salt, no CBL activity could be found using methionine as a substrate. However, significant CBL activity could be observed using cystathionine as a substrate, indicating that, at mimicked cheese preparation conditions, the production of homocysteine from L-cystathionine is catalysed by CBL, rather than the production of methanethiol from L-methionine.

Thus, at cheese preparation condition, CBL was not believed to contribute to the flavour formation during the cheese preparation or ripening proces. According to Smit et al (Food Res. Int. 32 (2000) pp 153-160), methanethiol can also be produced from methionine, independent from the action of CBL; via a transamination reaction, methionine can be converted in starter cultures into 4-methylthio-2-oxobutyrate (KMBA) and further decarboxylated to 3-methyl-thiopropionaldehyde (methional). Both KMBA and methional are described to be enzymatically converted in the starter cultures into methanethiol. Thus, the flavour formation in cheese preparation and ripening was believed to be controlled by other enzymes, rather than by CBL.

The cheese ripening process is slow, as the required enzymes are released from the lactic acid bacteria after permeabilisation or lysis thereof. Therefore, the ripening material, leading to the cheese product once the ripening procedure is completed, is to be stored for a considerable time at controlled conditions, which is an important cost factor.

Therefore, attempts have been made to accelerate the ripening process by e.g. adding enzyme preparations enhancing the digestion of proteins and cleavage of the peptides, and by using lactic acid bacteria having enhanced permeabilisation or lysis characteristics. Such bacteria become permeable or lyse, respectively, within a shorter period than the conventional lactic acid bacteria, so that the ripening process is accelerated.

The present inventors very surprisingly found that cheese products having enhanced and/or altered taste and aromatic properties could be produced by using bacteria that produce CBL in substantially higher amounts (i.e. having a substantially higher CBL activity) than the amounts (i.e. CBL activity) produced by the starter cultures, presently known in the art. Further, it was found that lysis of the bacteria in cheese was not necessary to obtain the improved and/or altered taste. The invention thus relates to a method for the preparation of a cheese product, comprising the step of adding bacteria having cystathionine-β-lyase (CBL) activity to material from which the cheese product is formed, wherein the bacteria, when added to cheese milk in an amount of up to 1 X 10⁷ bacteria per ml cheese milk, have a specific CBL activity of at least 40 nmol mercaptide produced per ml cheese milk per minute.

With "altered taste", mainly a so-called sulphur and thermophilic taste (sweet, brothy and fruity) is meant. According to the invention, this sulphur and thermophilic taste can be enhanced or provided in cheese products. The method according to the invention therefore also relates to a method for the preparation of a cheese product having improved and/or altered taste comprising the step of adding bacteria as defined above.

"Material from which the cheese product is formed" is defined as any material used in the cheese preparation process, which itself, or a product converted therefrom, is present in the resulting cheese product. Examples of such materials is the starting material milk and any intermediate product such as curd or cheese in the process of being ripened, such as e.g. curd cheese or cheddar cheese, although curd cheese or cheddar cheese may as well be the final cheese product to be produced by the method of the present invention. The ripened cheese may however also be regarded as "material from which the cheese product is formed", e.g. in case processed cheese products are to be prepared by adding bacteria to the ripened cheese, e.g. after a heating step. According to the invention, the bacteria may therefore be added to the starting material cheese milk, or to any intermediate products such as curd, or to the cheese after ripening. The bacteria may also be added at different time points in the cheese preparation process, e.g. to the starting material (such as cheese milk) and to an intermediate product.

Herein, "specific CBL activity" is defined as units thiol, such as mercaptide, generated/mg protein per minute using cystathionine as a substrate in the method described by J.R. Uren (Methods Enzymology, 1987, vol. 143, pp. 483-486). The specific activity is measured according to the method described by J.R. Uren, supra, wherein, instead of measuring the activity from cell extracts, the said activity is measured from cheese milk.

As outlined above, the bacteria are not necessarily added to the cheese milk but the bacteria will have the above defined CBL activity when added to cheese milk at cheese preparation conditions, which are known in the art.

The bacteria can constitute a mixture of different bacterial strains, as long as the said mixture as a whole has the above identified specific CBL activity. In addition to the said bacteria or bacteria mixture, additional different bacteria can be added during the cheese preparation process.

The bacteria having the above identified specific CBL activity are herein also referred to as "CBL overproducing bacteria". Bacterial cultures, used in the art in the cheese preparation process have a very low CBL activity, i.e. below 10 mercaptide formed per ml cheese milk, when added in an amount of up to 10⁷ cells per ml cheese milk.

By adding CBL overproducing bacteria in the cheese preparation process, an increased amount of CBL activity will become available in the material from which the cheese product is formed, therewith resulting in a cheese product of improved taste and having altered aromatic properties. Further, the ripening process may be accelerated.

It has been found that the taste of the cheese improves even further, in particular regarding thermofilic and sulphur flavour, when bacteria are added according to the invention, having an even more increased specific CBL activity. Bacteria, having a specific CBL activity in the cheese milk of as high as 1840 nmol mercaptide produced per ml cheese milk per minute, based on 9 x 10⁶ bacteria per ml cheese milk, resulted, when added to the cheese milk in the cheese preparation process, to clear additional thermophilic and sulphur flavour, even after shorter ripening periods. Therefore, in a special embodiment of the invention, the specific CBL activity of the bacteria added in the cheese preparation process according to the present invention is at least 100 nmol, preferably at least 200 nmol, more preferably at least 500 nmol, even more preferably at least 1000 nmol, most preferably at least 1800 nmol mercaptide produced per ml cheese milk per minute, when the bacteria are added to the cheese milk in an amount of up to 1 x 10⁷ bacteria.

In order to obtain a cheese product having optimally improved taste and flavour, at least 10⁶, preferably between 2.0 - 4.0 x 10⁶ of the CBL overproducing bacteria are, per ml cheese milk, added to the material from which the cheese product is formed. In case the bacteria are not added to cheese milk, but during another stage of the cheese preparation process, the preferred amount of CBL overproducing bacteria is based on the volume of cheese milk, used in the particular cheese preparation process.

However, according to the present invention, preferably at least a portion of the bacteria is added to the cheese milk, most preferably, substantially all the CBL overproducing bacteria are added to the cheese milk. "Substantially all" is meant to encompas at least 90% of the CBL overproducing bacteria, added during the cheese preparation process. Most preferably, 98-100% of the CBL overproducing bacteria are added to the cheese milk. Preferably at least 10⁶, more preferably between 2.0 - 4.0 x 10⁶ CBL overproducing bacteria are added per ml cheese milk.

In a special embodiment of the invention, the CBL overproducing bacteria are combined with bacteria that do not, or hardly not, at least do not overproduce, CBL activity; such bacteria are defined as having a specific CBL activity of at most 20 nmol mercaptide produced per ml cheese milk per minute, when added to cheese milk in an amount of up to 1 x 10⁷ bacteria per ml cheese milk. Such bacteria are also referred to herein as "CBL-non-overproducing bacteria".

Thus, CBL overproducing bacteria can be combined with a starter culture known in the art; it has been found that by combining such a starter culture with a CBL overproducing bacterial strain, cheese products can be obtained as can be produced by the regular starter cultures, having however the improved taste and flavour characteristics as discussed above. It has been found that such cheese products can be obtained when relatively large amounts of the CBL overproducing bacteria are combined with the non-CBL overproducing bacteria, used as starter culture. Thus, in a very attractive embodiment of the invention, the added bacteria comprise a first number of a first group of bacteria, having, when added to cheese milk in an amount of up to 1 x 10⁷ bacteria per ml cheese milk, a specific CBL activity of at most 20 nmol mercaptide produced per ml cheese milk per minute, and a second number of a second group of bacteria, having, when added to cheese milk in an amount of up to 3 x 10⁶ bacteria per ml cheese milk, a specific CBL activity of at least 60 nmol mercaptide produced per ml cheese milk per minute, wherein the ratio of the first number to the second number is between 1.5 - 5.0 : 1, preferably 1.8 - 3.0 : 1, most preferably 1.9 - 2.5 : 1. However, it is also possible to add only CBL overproducing bacteria in the method according to the invention.

The above-mentioned ratio's are particularly preferred when the bacteria are added in the curding step.

However, it is also possible to use a relative higher amount of CBL overproducing bacteria. In particular, in the preparation process of processed cheese from curd or from cheese (the ripened curd), the said ratio may be substantially higher; the process can even, and advantageously, be carried out by the addition of CBL overproducing bacteria in the absence of non-CBL-overproducing bacteria. In the case of the production of a processed cheese product from curd or cheese according to the present invention, the process for the preparation of the said curd or cheese may however involve the addition of common CBL-non-overproducing bacteria, optionally combined with CBL overproducing bacteria.

In the above, when the preparation process involves both the addition of CBL overproducing bacteria and of non-CBL-overproducing bacteria, the indicated amounts and ratios therebetween are to be regarded as the respective amounts/ratios of added bacteria in the same step of the cheese preparation process. Addition of the non-CBL-overproducing can be simultaneous with the recombinant CBL overproducing bacteria, e.g. as a mixture; however, both CBL-non-overproducing and CBL-overproducing bacteria may also be added subsequently to one another within in a short time period within the same stage of the preparation process, where the order of addition may not be critical and can be determined by the skilled practitioner.

It is however also possible to add bacteria in more stages during the cheese preparation process; in that case, in each stage both CBL overproducing bacteria and CBL-non-overproducing bacteria can be added. As is outlined above, it is even possible to add CBL overproducing bacteria without addition of non-CBL-overproducing bacteria in one or more stages of the cheese preparation process.

By combining both CBL overproducing bacteria and CBL-non-overproducing bacteria, the production of CBL activity during the cheese preparation process, and therewith the taste and flavour development, can be adjusted.

As will be recognized from the above, the second group of bacteria will provide the CBL overproducting bacteria. In line with the above, the specific CBL activity of the said CBL overproducing bacteria of the second group is preferably at least 100 nmol, more preferably at least 200 nmol, even more preferably at least 500 nmol, even more preferably at least 1000 nmol and most preferably at least 1800 nmol mercaptide produced per ml cheese milk per minute, when the said CBL overproducing bacteria of the second group are added in an amount of up to 3 x 10⁶ bacteria per ml cheese milk.

Preferably, the CBL activity of the bacteria of the second group is at least 10 times, preferably at least 20 times the specific CBL activity of the bacteria of the first group.

Preferably, per ml cheese milk used for the preparation of the cheese product, at least 10⁶, more preferably between 2.0 - 4.0 x 10⁶ bacteria of the second group are added to the material from which the cheese product is formed. Preferably, the said bacteria of the second group are added to the cheese milk; however, the said bacteria can at least partially be added during a later stage of the cheese preparation process. The indicated amounts are based on the volume of cheese milk used to produce the envisaged cheese.

Further "helper" strains can be used, that themselves do not show improved production of CBL activity, or may not even be involved in the common cheese preparation process, but may produce agents required for improving the growth of and/or the induction of CBL production or release in the CBL overproducing bacterial strain.

In a very attractive embodiment of the invention, the second group of bacteria comprises at least one CBL overproducing recombinant bacterial strain.

"Recombinant CBL overproducing bacterial strain" is defined as a bacterial strain being genetically engineered such that it produces more CBL activity than the parent strain in which the genetic engineering has been carried out. The bacterial parent strain may as well be a genetically engineered recombinant strain, however, it is to be understood that said genetic engineering in the bacterial strain has preferably not led to an increase of production of CBL activity. Such a genetic engineered parent strain may e.g. be genetically engineered to obtain other characteristics than overproduction of CBL activity.

Said recombinant CBL overproducing bacterial strain preferably has a specific CBL activity of at least 40 nmol, preferably at least 60, more preferably at least 100, more preferably at least 200, more preferably at least 500, more preferably at least 1000 and most preferably at least 1800 nmol mercaptide produced per ml cheese milk per minute, when the said recombinant CBL overproducing bacterial strain is added to cheese milk in amount of up to 3 x 10⁶ bacteria per ml cheese milk.

The recombinant CBL overproducing bacterial strain may comprise its original CBL coding sequence, the *metC* gene, being operably linked to genetic elements that improve the expressing of the said endogenous *metC* gene. Said elements are known in the art and comprise e.g. promoter and of/or enhanced sequences. The overproducing strain may however comprise CBL encoding nucleic acid sequences, not present in the parent strain. Said additional encoding sequences may be derived from the same bacterial species, but may also be of heterologous origin, such as from Lactobacilli species and *Streptococcus thermophilus.*

Also, the CBL-non-overproducing bacteria may comprise recombinant bacteria, as long as the said bacteria do not produce, in cheese milk at cheese preparation conditions, a specific CBL activity of more than 40 nmol, preferably not more 20 nmol mercaptide produced per ml cheese milk per minute, when the said bacteria are added to cheese milk in an amount of up to 1 x 10⁷ bacteria.

Such a strain may e.g. be a recombinant, wherein the genetically engineered sequences do not substantially contribute to enhancement of the CBL production of the said bacteria. By combining both a recombinant CBL overproducing strain and a CBL-non-overproducing strain, either recombinant or not recombinant, the production of CBL activity during the cheese preparation process can be adjusted.

The added CBL overproducing bacteria preferably belong to a lactic acid bacteria strain but may also be of another species, such as e.g. a *Brevibacterium* strain, or of a bacterial strain that is not involved in the common cheese preparation process. However, it is important that such a bacterial strain is non-pathogenic and food-grade, having the co-called "GRAS"(generally recognised as safe)-status. Preferably however, all the bacteria used in the method according to the present invention, i.e. optionally including the recombinant CBL overproducing bacteria, are bacteria, involved in the common cheese preparation process, and are most preferably lactic acid bacteria.

The expression of the CBL from the recombinant CBL overproducing bacterial strain is preferably induced. Thus, the recombinant strain can be made to overproduce CBL activity by controlling the conditions wherein the CBL expression is induced. In the art, inducible expression systems are well known; e.g., for Lactococci, the *rlt* system is described in Nauta et al, 1997, Nature Biotechnology, vol. 15, pp. 980-983 and may be used according to the invention. Preferably, the CBL expression from the recombinant CBL overproducing bacterial strain is inducible by nisin. An inducible heterologous gene expression system, inducible by nisin is known from EP 0712935, under the trade name NICE™. In summary, the heterologous gene, in the present case a gene encoding CBL activity, is operably linked to the nisin promoter. The expression under control of the nisin promoter increases with the amount of nisin, available to the bacteria. In a special embodiment, an additional inducer producing bacterial strain, such as in this case a nisin producing strain, such as *L. lactis* subspecies. lactis var. *diacetylactis* TC 17-5 (commercial NIZOSTAR culture of CSK food enrichment, Leeuwarden, The Netherlands), is added in the cheese preparation process, so that sufficient inducer is present for induction of the CBL encoding sequence on the recombinant CBL overproducing bacterial strain.

In an attractive embodiment, the expression of CBL from the recombinant CBL overproducing strain is induced before adding of the said strain to the material from which the cheese product is formed. When the recombinant strain is already induced before adding in the cheese preparation process, the CBL gene expression is already switched on and the recombinant bacteria are able to overproduce CBL activity directly after adding thereof in the process, therewith avoiding the phase of low CBL production, during induction process. The recombinant bacteria may already overproduce CBL before being added in the cheese preparation process, e.g. in the preceding injection step, so that in combination of already induced recombinant bacteria as well as already produced CBL is simultaneously added in the cheese preparation process.

It may be advantageous when the material from which the cheese is formed comprises an agent, enhancing the release of CBL from the CBL overproducing bacteria in the material from which the cheese product is formed. Such an agent may induce the permeability of the bacterial membrane, so that the CBL molecules can "leak out" of the cell, or may enhance lysis of the CBL overproducing bacteria. By the presence of such a lytic agent, the CBL overproducing bacteria may be more susceptible to lysis, which may lead to a better availability of CBL in the material, therewith enhancing the cheese ripening process which may result into improvement of cheese taste and of decreasing the period needed for cheese ripening and therewith storage time; this may in particular be true for the production of cheese paste. The release enhancing agent may be added during the cheese preparation process in the form of a compound formulation or may be produced by bacteria or other micro-organisms that may be added during the cheese preparation process. It is also possible that e.g. the CBL-overproducing bacteria themselves are capable to produce a lytic agent, leading to lysis of the cells. Such endogenous production of a lysis enhancing agent may also be provided by an inducible expression system or may be produced through constitutive expression of a gene encoding such a lysis enhancing agent. In a preferred embodiment however, the CBL release enhancing agent, preferably a lytic agent, is induced by an additional bacterial strain that is added to the material from which the cheese product is formed. In the said strain the expression of the gene encoding the lysis enhancing agent may also be constitutively or inducibly be expressed.

Preferably, the recombinant CBL overproducing strain is *Lactococcus lactis* NZ3900, wherein a CBL-encoding nucleic sequence is operably linked to a high expression promoter, functional in the said bacteria. NZ3900 is chosen as a host since it carries in the *pepN* locus the *nisR* and *nisK* genes, required for signal transduction of nisin (Kuipers et al, 1993. Eur. J. Biochem., vol. 216, pp. 27299-27304). In addition, NZ3900 carries a deletion in the chromosomal *lacF*-gene and therefore cannot grow on lactose (de Ruyter et al., 1996, J. Bacteriol., vol 178, pp. 3434-3439). Transformation of NZ3900 with plasmid pNZ8143 harbouring the food-grade *lacF* marker restores the ability to grow on lactose as a sole carbon source (Platteeuw et al, 1996, Applied and Environmental Microbiology, vol. 62, pp. 1008-1013). The parent strain of *Lactobacillus lactis* NZ3900, NZ9000, is described in N. Fernandez et al, Applied Microbiology 66:42-48(2000). Extracts of *L. lactis* cells of strain NZ9000, harbouring pNZ8137, which overproduce CBL, were shown to form larger amounts of sulphur compounds from methionine than the wild-type strain in an *in vitro* assay (Fernandez et al, 2000, Applied Microbiology 66:42-48).

Preferably, the recombinant CBL overproducing bacterial strain comprises one or more copies of a plasmid chosen from pNZ8140 (deposited at the Centraal Bureau voor Schimmelcultures (CBS), P.O. Box 85167, Utrecht, The Netherlands on december 13, 2000 according to the requirements of the Budapest Treaty under accession number CBS 109210) and pNZ8143(deposited at the CBS on december 13, 2000 according to the requirements of the Budapest Treaty under accession number CBS 109211). Plasmid pNZ8143 is a derivative of plasmid pNZ8137 (Fernandez et al, 2000), in which the Cm^{R}-gene is replaced by the lacF gene as a food-grade selectable marker. Furthermore, a stretch of 60 bp *Bacillus subtilis* DNA is removed from the original plasmid. The plasmid further carries the pHS71 replicon and the *L. lactis* B78 *metC* gene, encoding cystathionine-β-lyase, under the control of the nisin promoter. The resulting plasmid pNZ8143 consists only of L. lactis DNA and can therefore be regarded as a food-grade vector (see Lindgren (1999), Int. Dairy J. vol. 9 pp. 37-41; see also example 2)

In a preferred embodiment, the invention also relates to the novel plasmids pNZ8140 and pNZ8143, which are extremely useful for the preparation of recombinant bacteria, capable of overproducing CBL.

The invention further relates to a cheese product, obtainable by the method according to the invention, the said cheese product having a specific CBL activity of at least 20 nmol, preferably at least 60 nmol and most preferably at least 80 nmol mercaptide, produced per g cheese product per minute. The specific CBL activity from cheese is measured according to the method of J.R. Uren, supra, wherein the CBL activity is measured from cheese extracts, prepared according to example 4, below, rather than from cell extracts. Such a cheese product may be produced using CBL overproducing bacteria, resulting in the relatively high specific CBL activity in the obtained cheese product. Alternatively, purified CBL can be added during the cheese preparation process, e.g. to the cheese milk. Cheese, prepared using starter cultures, known in the art have a specific CBL activity in the range of 3-4 nmol mercaptide formed per g cheese per minute.

In a further aspect, the invention relates to cheese milk, having a specific CBL activity of at least 100 nmol, preferably at least 200 nmol, more preferably at least 500 nmol, more preferably at least 1000 nmol, most preferably at least 1800 nmol mercaptide produced per ml cheese milk per minute. Said cheese milk, having such a high specific CBL activity is optimally suited for the preparation of a cheese product having enhanced taste and flavour characteristics, as discussed above.

The invention will be further illustrated by the following examples and figures, wherein
Figure 1 shows a schematic diagram of a common cheese preparation process,
Figure 2 shows a scheme of the cloning strategy for the construction of pNZ8140, and
Figure 3 shows plasmid maps of pNZ8140, pNZ8141, pNZ8142 and pNZ8143.
In the scheme of figure 1, the common cheese preparation process is illustrated. Starting material is cheese milk, which can be any kind of milk, such as cow milk, goat milk, sheep milk, etcetera.

In a first step, also indicated as curding step, and indicated with arrow 1, bacteria and/or enzymes (amount and species depends on the kind of cheese to be obtained) are added to the cheese milk, resulting to curd. During step 1, CBL overproducing bacteria may be added to the cheese milk, with or without the addition of CBL-non-overproducing bacteria such as a common starter culture, comprising lactic acid bacteria.

The second step (arrow 2) is the ripening stage, wherein the curd is ripened to cheese by storing the curd at the proper conditions, depending on the kind of cheese to be produced. In the ripening stage, usually no bacteria are added. The invention however also encompasses variants of the cheese preparation process, wherein CBL-overproducing bacteria are added to the curd during the ripening stage.

From the cheese, but also from the curd, cheese based products can be produced by a third processing step (arrows 3a and 3b, respectively), comprising heating the cheese or curd, followed by the addition of bacteria and/or enzymes. This third processing step is often done in countries where processed or cheese analogues are popular.

According to the invention, in each of the steps, the CBL overproducing bacteria can be added, with or without addition in the said same step of CBL-non-overproducing bacteria. When, in case of the preparation of cheese based products, bacteria are added in two different stages (stages 1 and 3), in one or each of the said stages CBL overproducing bacteria can be added, with or without addition of non-CBL-overproducing bacteria; e.g., in step 1 both CBL overproducing bacteria and CBL-non-overproducing bacteria can be added, whereas, later in step 3 of the process, only CBL overproducing or only CBL-non-overproducing bacteria can be added. However, in a special embodiment of the invention, in step 3 CBL overproducing bacteria are added, without the addition of CBL-non-overproducing bacteria.

### Example 1

### Food-grade overproduction of CBL using NICE™-system

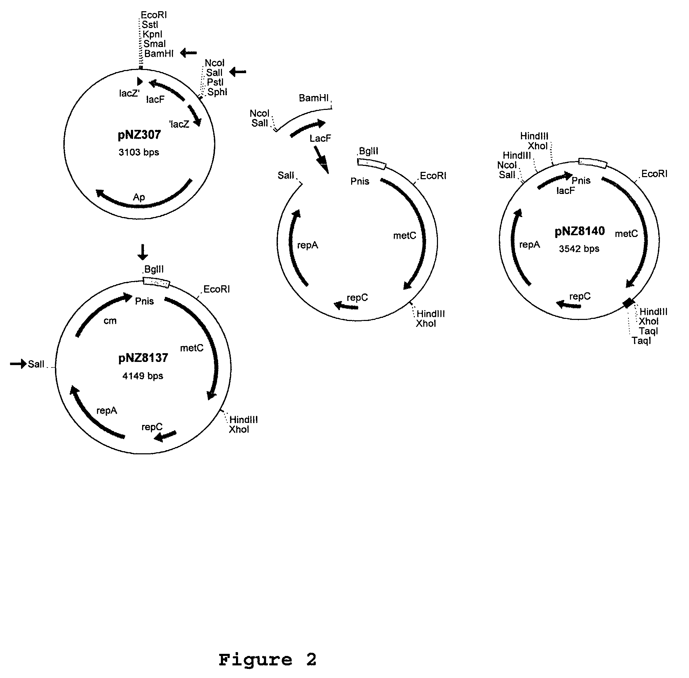

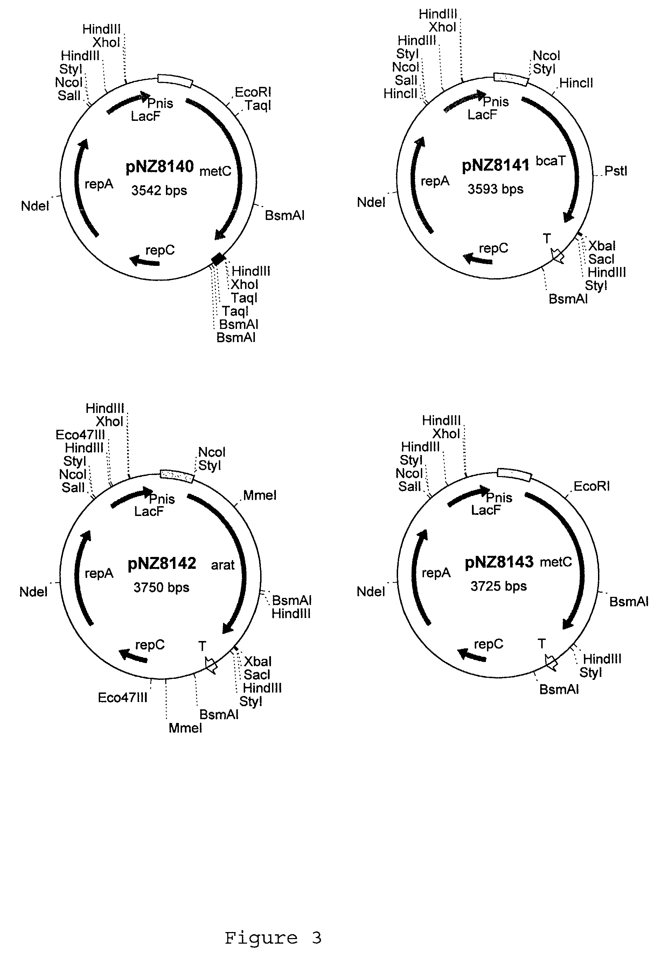

To demonstrate that cystathionine β-lyase (CBL) can be overproduced in a food-grade manner in *L. lactis* using the NICE™ system, the below mentioned procedure for construction of plasmids was followed. In this study the methionine-converting enzyme CBL of *L. lactis* strain NIZO B78 was expressed with the NICE™ system. For this purpose a food-grade plasmid was constructed that make use of the food-grade selection marker *lacF*. Plasmid pNZ8137 contains the *metC* gene, coding for cystathionine β-lyase, of *L. lactis* strain B78 cloned under control of the *nisA* promoter of pNZ8037 (M. Fernandez et al., 2000 Appl. Environ. Microbiol., vol. 66, 42-48).
A food-grade derivative, pNZ8140, was constructed by replacement of the *cat* gene encoding chloramphenicol resistance by the food-grade marker *lacF* (Figure 2). Expression of the promoter-less *lacF* gene is obtained by the readthrough from the *repA* gene. Careful analysis of the complete sequence of plasmid pNZ8140 showed that it contains a stretch of 60 bp originating from *Bacillus subtilis.* Although this is not supposed to harm the self-cloning status of the plasmid, it was decided to construct a new cloning vector without the *B. subtilis* DNA-fragment that, consequently, only consists of *L. lactis* DNA. Therefore, a fragment containing this 60-bp fragment was deleted from the NICE™ plasmid pNZ8048 resulting in plasmid pNZ8148. The *metC* gene was cloned from plasmid pNZ8137 into pNZ8148 yielding pNZ8148-*metC*. Subsequently, the *cat* gene was replaced by *lacF* resulting in the food-grade plasmid pNZ8143 (Figure 3; therein, terminators are indicated by an open arrow. The *B. subtilis* sequence in pNZ8140 is indicated as a black box).
Plasmid pNZ8143 was transformed into strain *L.lactis* NZ3900 (de Ruyter et al., 1996, J. Bacteriol. vol. 178, pp. 3434-3439). NZ3900 is chosen as a host since it carries in the *pepN* locus the *nisR* and *nisK* genes, required for signal transduction of nisin (Kuipers et al.,1993, Eur. J. Biochem., vol, 216, 27299-27304). NZ3900 also carries a deletion in the chromosomal *lacF* gene and therefore cannot grow on lactose (de Ruyter et al., 1996, J. Bacteriol. vol. 178, pp. 3434-3439). Transformation of NZ3900 with plasmid pNZ8143 harbouring the food-grade lacF marker restores the ability to grow on lactose as a sole carbon source (Platteeuw et al., 1996, Appl. Environ. Microbiol, vol. 62, 1008-1013).
For CBL overproduction with the NICE™ system, *L. lactis* NZ3900 harboring pNZ8143 was cultured in M17 medium supplemented with 0.5% lactose to an optical density at 600 nm (OD₆₀₀) between 0.5 and 0.7, induced with 0, 0.1, or 1.0 ng nisin A per ml and incubated for another 2 hours at 30°C. Cell-free extracts were prepared and CBL activity towards cystathionine was measured by determination of free thiol group formation with DTNB as described by Uren (Methods in Enzymology, 1987, vol. 143, pp. 483-486) using 13200 as the extinction coefficient to calculate the results.
The cystathionine-β-lyase (CBL) activity was determined for the cell-free extracts of the cultures overproducing CBL (Table 1). Induction with 1 ng nisin/ml resulted in a 23-fold increase in CBL activity compared to the uninduced culture. As a control *L. lactis* NZ9000 harbouring pNZ8137 was used (M. Fernandez et al., 2000. Appl.
Environ. Microbiol., vol. 66, 42-48). The specific activity of this strain was 2.0 for an uninduced culture and 273 for the culture induced with 1.0 ng nisin per ml. The specific CBL activity of *L. lactis* NIZO B78 grown in milk is 14 nmol per mg protein per minute (A. C. Alting et al.,1995. Appl. Environ. Microbiol., vol. 61, 4037-4042). The CBL activity of strain NZ9000 is more than 10-fold lower than that of NZ3900 harbouring pNZ8143, while strain B78 has an intermediate activity. The difference in CBL activity between strain NZ3900 and NZ9000 may be due to the fact that these host strains are not identical (the difference is that NZ3900 contains a chromosomal integration of the lactococcal lactose operon (with *lacF* deleted)). Nevertheless, these results show that a 50-fold overproduction of CBL specific activity is achieved in fully induced cells of NZ3900 carrying pNZ8143 when compared with the natural wild-type strain *L. lactis* NIZO B78.

**Table 1.**

| Specific and relative CBL activities of *L. lactis* NZ3900 harbouring pNZ8143. | | |
|---|---|---|
| Concentration of nisin A (ng/ml) | Specific Activity (nmol of mercaptide formed per mg protein per min) | Relative Activity |
| 0.0 | 30 | 1.0 |
| 0.1 | 46 | 1.5 |
| 1.0 | 690 | 23.0 |

### Example 2

### The effect of CBL overproduction on flavour development in cheese paste.

Current legislation does not allow the cheese graders to taste a cheese paste with high inoculation levels of *L. lactis* NZ3900 harbouring pNZ8143, because this strain is still considered as a genetically modified microorganism. Therefore, to study the effect of CBL overproduction on flavour development in a cheese paste permeabilised cells were used. With this approach the enzymes are retained inside the cell, while the substrate molecules and flavour compounds can diffuse freely into or out of the cell. Therefore, flavour formation will not depend on active transport of substrate molecules into the cell and diffusion of the flavour compounds to the cheese paste.
Firstly, it was tested whether this method could be used for CBL, by comparison of CBL activity of cell-free extracts made from the same culture of *L. lactis* NZ3900 harboring pNZ8143 that were either permeabilised with various concentrations of butanol or untreated. Cells from a log phase-culture were divided in two batches. One was incubated with 5% butanol for permeabilisation. The butanol was removed by washing 5 times with a buffer and the cells were used to make cell-free extracts. The other batch was not treated with butanol and immediately used to make cell-free extracts. Treatment with 5% butanol did not effect the CBL activity (Table 2), while it reduced the amount of living cells approximately 10⁹-fold compared to a reduction of 10-fold for 3% butanol (data not shown).

**Table 2.**

| Effect of permeabilisation on CBL activity. | | | | |
|---|---|---|---|---|
| | control | 3 % butanol | 4 % butanol | 5 % butanol |
| Relative CBL activity | 1 | 1.15 | 0.94 | 0.98 |
| CBL activity in cell-extracts *of L. lactis* cells (control), cells permeabilised with 3, 4 and 5 % butanol relative to that of non-permeabilised cells. | | | | |

In this experiment the CBL activity of the induced cells was approximately 12 times higher than that of the uninduced cells. The cheese pastes were inoculated in duplicate with water (blanc), ~10⁹ cells/gram paste of *L. lactis* NZ3900 harbouring pNZ8143 precultured in LM17, or ~10⁹ cells/gram paste of *L. lactis* NZ3900 harbouring pNZ8143 precultured in LM17 and induced with 2 ng nisin per ml culture. The cheese pastes were incubated at 13°C for 3 weeks after which one series was rated by 9 persons for aroma and taste and the other was used for sulphur analysis using HS-GC. In addition, a cheese paste was inoculated with ~10 ⁹ cells/gram paste of *L. lactis* NZ3900 which were not treated with butanol. This latter cheese paste was only subjected to GC-analysis, because graders were not allowed to taste a cheese paste with untreated cells (see above).
After 3 weeks incubation, the cheese paste containing the induced cells was unanimously graded as having more Gouda flavour and was sometimes judged as more thermophilic (sweet and broth). In addition, this cheese paste harbored a sulphur-flavour note as judged by the graders (Table 3). In contrast, the cheese with the uninduced cells was very much like the water-supplemented cheese paste and was only judged to be less flat. This result shows that overproduction of cystathionine β-lyase not only accelerates Gouda flavour development, but in addition produces an extra sulphur/thermophilic flavour in a cheese paste.

**Table 3.**

| Grading scores of cheese pastes after 3 weeks incubation at 13°C. | | |
|---|---|---|
| | Gouda flavour | Sulphur/thermophilic-flavour intensity |
| blank, no cells added | 6.0 | 0 |
| non-induced cells (permeabilised) | 6.2 | 0 |
| nisin-induced cells (permeabilised) | 6.6 | 0.3 |
| Scales: aroma/taste Gouda from 4-8, and thermophilic/sulphur from 0-4. | | |

HS-GC analysis of the cheese pastes (Table 4) confirmed the findings of the grading. The cheese paste incubated with induced cells showed a significant increase in amount of volatile sulphur compounds methanethiol (0.5 -fold), dimethylsulfide (3-fold) and dimethyldisulphide (3-fold) when compared with uninduced cells. In contrast, the amount of carbondisulfide remained constant because this compound is not produced by the action of cystathionine β-lyase.

Furthermore, treatment with butanol of induced cells increases the amount of above mentioned volatiles in the cheese paste with 30-50 %, indicating that a permeabilised cell membrane enhances sulphur-compound formation produced by the enzyme cystathionine β-lyase in the cheese paste.

**Table 4.**

| Sulphur compounds detected in cheese pastes. | | | | |
|---|---|---|---|---|
| cells | methanethiol (ppb) | dimethylsulfide (ppb) | dimethyldisu lfide (ppb) | carbonsulfide (ppb) |
| permeabilised uninduced | 29500 | 12000 | 7500 | 5000 |
| permeabilised induced | 44500 | 50500 | 30500 | 6000 |
| non-permeabilised induced | 29000 | 26000 | 20500 | 5500 |
| Sulphur compounds detected in cheese pastes incubated with permeabilised *L. lactis* cells overproducing CBL (induced), permeabilised cells (uninduced) and control (non-permeabilised, induced) cells. Methanethiol, dimethylsulfide, and dimethyldisulfide, are produced by methionine lyase activity, while carbondisulfides are not. | | | | |

### Example 3

### The effect of CBL overproduction on flavour development in Gouda-type cheese.

To establish the effect of the recombinant CBL-overproducing strain on flavour formation in cheese, Gouda cheese trials were performed. Gouda cheese was prepared from 200 L portions of pasteurized milk (10 sec., 74 °C) according to standard Gouda cheesemaking procedures (Walstra, P,, Noomen, A. & Geurts, T.J. (1987) Dutch-type varieties. In P.F. Fox, Cheese: chemistry, physics and microbiology, pp. 45-92, Elsevier, London). The traditional Bos cheese starter culture was cultivated in pasteurised skimmed milk for 16 hours at 18 °C. Culture NZ3900 harboring pNZ8143 (CBL overproducer) was grown in LM17-broth and induced with nisin as indicated in example 1. The nisin-producer TC17-5 (commercial CSK culture, Leeuwarden, The Netherlands) was added to the cheesemilk by direct inoculation from the concentrate culture. The used culture dosages in cheese trials are indicated in Table 5. Since the dosage of the nisin-producing culture to the cheese milk is very low, nisin concentrations in cheese are subinhibitory and no effect was observed on the acidification rate during cheese making. After brining the cheeses were ripened at indicated periods at 13 °C en 85 % relative air moisture.

**Table 5.**

| Culture dosage in percentage (v/v). | | | | |
|---|---|---|---|---|
| trial | BOS (% v/v) 10⁹ cells/ml | NZ3900 +pNZ8143 (% v/v) 3 x 10⁸ cells/ml | TC17-5 (% v/v) 10⁹ cells/ml | Specific activity CBL (umol mercaptide formed per ml. cheese milk per min.) |
| 1 | 0.6 | - | 0.005 | 0.008 |
| 2 | 0.6 | 1 (non-induced) | 0.005 | 0.064 |
| 3 | 0.6 | 1 (induced) | 0.005 | 1.840 |
| 4 | 0.6 | 1 (induced) | - | 1.840 |

The BOS starter culture was present in an amount of 6 x 10⁶ cells/ml cheese milk; NZ3900, harbouring plasmid pNZ8143 was present in the cheese milk in an amount of 3 x 10⁶ cells per ml; TC17-5 was present in an amount of about 5 x 10⁴ cells/ml. Thus, the total number of cells in the cheese milk is about 9 x 10⁶ cells/ml cheese milk if both BOS and NZ3900 (harbouring pNZ8143) are present. The addition of the above strains to the cheese milk results in a total CBL-activity as indicated. The specific activities of 0.008, 0.064 and 1.840, expressed as nmol mercaptide formed per ml cheese milk per minute correspond to a specific activity, expressed in nmol mercaptide formed per mg protein per minute of 2.1, 18.9 and 592.1 respectively. The contribution of CBL activity of strain TC17-5 is neglectable in comparison with that of BOS and NZ3900.

The cheeses were graded by a trained panel consisting of 8 persons at 6, 13 and 16 weeks of ripening. At 6 weeks of ripening the graders did not observe any organoleptical differences between the cheeses of various cheese trials. The gradings results at 13 and 16 weeks of ripening are shown in Table 6.

**Table 6.**

| Grading results of Gouda cheese prepared with various culture dosages (see Table 5). | | | | | | | | |
|---|---|---|---|---|---|---|---|---|
| | 13 weeks ripening | | | | 16 weeks ripening | | | |
| | 1 | 2 | 3 | 4 | 1 | 2 | 3 | 4 |
| aroma/ taste | 6.2 ± | 6.2 ± | 6.5 ± | 6.2 ± | 6.3 ± | 6.4 ± | 6.3 ± | 6.5 ± |
| | 0.4 | ± 0.5 | ± 0.4 | ± 0.3 | 0.3 | 0.3 | 0.3 | 0.5 |
| Thermophilic/ sulphur | 0.2 ± | 0.2 ± | 0.3 ± | 0.5 ± | 0.2 ± | 0.7 ± | 0.8 ± | 0.9 ± |
| | ± 0.2 | ± 0.3 | ± 0.4 | ± 0.4 | 0.3 | 0.2 | 0.3 | 0.3 |
| Scales: aroma/taste from 4-8, and thermophilic/sulphur from 0-4. | | | | | | | | |

After 13 weeks ripening a trace of thermophilic/sulphur flavour is observed in cheeses prepared with NZ3900 harboring pNZ8143 (trial 3 and 4). The overall score for aroma and taste was similar for all cheeses.

At 16 weeks ripening the graders observed a clear additional flavour, specified as thermophilic and sulphur, in particular in cheeses prepared in trials 3 and 4. Since no significant flavour differences were observed between cheeses 3 and 4, this indicates that in the case of cheese containing preinduced recombinant cells, the addition of an additional nisin-producing strain TC17-5 has no significant effect on flavour development.
However, in cheese prepared with a non-induced recombinant strain, the addition of the nisin-producing strain TC17-5 (trial 2) causes also an additional thermophilic/sulphur-aroma to the cheeses, but less in intensity in comparison with cheese of trials 3 and 4 (Table 6). It is believed that in trial 2 the inducer strain TC17-5 produces such amount of nisin in cheese that the NICE™-system is induced suboptimally and less CBL is overproduced in recombinant cells in comparison with fully pre-induced cells as applied in trials 3 and 4. Subsequently, this relatively reduced CBL activity in trial 2 results in less thermophilic/sulphur flavour development. In the European patent application EP 0712935 it is described that the level of induction of the NICE™-system is nisin dosage dependent.

Finally, the grading results indicates that the overall scores for aroma and taste at 16 weeks ripening for all cheeses are similar.

In summary, these results show that addition of a recombinant strain overproducing CBL having an at least 8-fold increased CBL activity as compared to that of the commonly used bacterial strains, to the cheese milk together with a traditional Gouda cheese starter (Bos), produces an extra thermophilic and sulphur flavour to the Gouda cheese after ripening, in particular at 16 weeks of ripening. Even better thermophic/sulphur flavour is obtained with CBL-overproducers, having a more than 100-fold, preferably more than 200-fold (in this particular case 230-fold) CBL activity as compared to that of commonly used bacterial strains. Thus the CBL overproducing strain is able to diversify Gouda cheese flavour development.

The effects of recombinant CBL strain on flavour development were clearly observed by the cheese graders after a ripening period of 16 weeks. This may be due to the fact that the recombinant strain NZ3900 carrying pNZ8143 might lyse relatively poor in the cheese matrix, thereby resulting in suboptimal early realisation of CBL activity towards its substrate methionine. This is confirmed by the cheese paste experiment which shows that permeabilisation of the cell membrane of strain NZ3900 carrying pNZ8143 enhances flavour development (example 2).

### Example 4.

### The effect of increased lysis of the CBL-overproducing strain on cheese flavour development.

Lysis of starter bacteria during cheese ripening results in the release of intracellular enzymes involved in proteolysis, lypolisis and amino acid degradation. These enzymes are involved in cheese ripening and enhancement of the rate of lysis could accelerate/alter flavour development in cheese (E. Ayad, 2001, PhD Thesis, Wageningen University; de Ruyter *et al*., Nature Biotechnology, 1997,15, pp. 976-979). Therefore, in order to establish whether lysis of the CBL-overproducing strain NZ3900 harbouring pNZ8143 is a limiting factor on cheese flavour development, we induced lysis of this strain, thereby liberating the overproduced CBL enzyme into the cheese matrix.

For this purpose, together with the CBL overproducing strain NZ3900 containing pNZ8143, another recombinant lactococcal strain NZ3900 containing plasmid pNZ8038F was added to the cheese milk. NZ3900 harbouring pNZ8038F produces the lysin and holin proteins of the lactococcal bacteriophage US3. The food-grade plasmid pNZ8038F is derived from pNZ8038 (de Ruyter *et al.,* Nature Biotechnology, 1997,15, pp. 976-979), whereby the selectable marker Cm^{r} of pNZ8038 is exchanged by the food-grade marker *lacF* gene (an approach similar as described in example 1). Simultaneous production of lysin and holin in cheese was shown to cause not only efficient lysis of intracellular enzymes of the adjunct itself containing pNZ8038, but also partial lysis of the surrounding bacteria (de Ruyter *et al.,* Nature Biotechnology, 1997,15, pp. 976-979).

Gouda cheese was manufactured as described in example 3. Strain NZ3900 harbouring pNZ8143 was cultivated and induced for CBL production with nisin as described in example 1. NZ3900 containing plasmid pNZ8038F was added to the cheese milk and induced for lysin and holin production as described by de Ruyter *et al*., Nature Biotechnology, 1997,15, pp. 976-979. Table 7 presents the dosages of the applied cultures in cheese trials, also including the specific activity of CBL in the cheese milk and in the manufactured cheese at 16 weeks of ripening. For determination of CBL-activity in cheese, 10 grams of cheese sample is mixed with 90 ml. of 2 % (w/v) sodium citrate at a temperature of 45°C. The cheese sample is homogenized for 5 minutes using a stomacher. The debris is removed from the suspension by centrifugation twice for 10 min. 10000 rpm at 5°C. CBL activity in the cheese extract (supernatant) was measured as described in example 1, according to J.R. Uren, supra, however in indicated cases the enzyme cofactor pyridoxal-5'-phosphate was omitted from the assay buffer.

**Table 7.**

| Culture dosage and CBL activity during cheese manufacturing. | | | | | |
|---|---|---|---|---|---|
| trial | BOS (% v/v) 10⁹ cells/ml | NZ3900 + pNZ8143 (% v/v) 3 x 10⁸ cells/ml | NZ3900 + pNZ8038F (% v/v) 3 x 10⁸ cells/ml | Specific activity CBL (umol mercaptide formed per ml. cheese milk per min.) | Specific activity CBL (umol mercaptide formed per gram cheese per min.) |
| 1 | 0.6 | - | - | 0.008 | 3.8 |
| 2 | 0.6 | 1 | - | 1.74 | 83.4 |
| 3 | 0.6 | 1 | 0.4 | 1.80 | 178.1 |

The addition of above strains to the cheese milk results in a total CBL-activity/ml cheese milk as indicated in Table 7. The total number of cells in the cheese milk with Bos and NZ3900 + pNZ8143 is 9 x 10⁶ cfu/ml. This number of cells is increased to a total of 13 x 10⁶ cfu/ml with the addition of strain NZ3900 + pNZ8038F (trial 3)

Our results show that addition of strain NZ3900 containing pNZ8143 to the cheese milk (trial 2) results in an increase of CBL activity in the cheese milk and cheese matrix with a factor 170 and 22, respectively, in comparison with the reference cheese (trial 1). Production of lysin and holin by NZ3900 harbouring pNZ8038F in the cheese induces cell lysis during cheese ripening and liberates the CBL enzyme from cells of strain NZ3900 containing pNZ8143: the CBL-enzyme activity in the cheese extract of trial 3 is increased with a factor 2 in comparison with the activity measured in trial 2.

The cheeses were graded by a trained panel consisting of 8 persons at 12 and 16 weeks of ripening. The grading scores (means) and standard deviations are presented in table 8.

**Table 8.**

| Grading results of Gouda cheese prepared with various culture dosages (see table 7) | | | | | | |
|---|---|---|---|---|---|---|
| trial | 12 weeks ripening | | | 16 weeks ripening | | |
| | 1 | 2 | 3 | 1 | 2 | 3 |
| aroma/taste | 6.54 ± | 6.36 ± | 6.69 ± | 6.26 ± | 6.25 ± | 6.63 ± |
| | 0.35 | 0.35 | 0.35 | 0.24 | 0.31 | 0.37 |
| thermophilic/ sulphur | 0.14 ± | 0.61 ± | 0.25 ± | 0.04 ± | 0.43 ± | 0.34 ± |
| | 0.15 | 0.48 | 0.25 | 0.10 | 0.71 | 0.40 |
| Scales: aroma/taste from 4-8, and thermophilic/sulphur from 0-4. | | | | | | |

As observed in example 3, cheese manufactured with a lactococcal strain overproducing CBL (trial 2) results in an increased score for thermophilic/sulphur flavour at 12 and 16 weeks of ripening in comparison with the reference cheese (trial 1). However, the results also show that enhanced lysis of the cells harbouring the overproduced CBL (trial 3), does not lead to an increased thermophilic/sulphur flavour in cheese in comparison with the reference cheese of trial 2.

The activity of the CBL-enzyme is dependent on the presence of its cofactor pyridoxal-5'-phosphate, which is noncovalently bound to the enzyme (Alting *et al,* 1995, Applied and Environmental Microbiology, 61, 4037-4042). Without deemed to be bound to any explanation, it could be speculated that lysis of the cells in cheese results in dilution of the enzyme cofactor, thereby rendering the enzyme inactive in the cheese matrix. To establish whether the released CBL enzyme is active *in vivo* in the cheese matrix, the activity of CBL in the cheese extract of trial 3 at 16 weeks ripening was determined in an assay in which pyridoxal-5'-phosphate was omitted from the assay buffer. Without being bound to any interpretation, the results indicated that CBL enzyme activity in cheese extracts was dependent on the presence of the cofactor in the assay buffer. The major part of the overproduced CBL enzyme may therefore be present in an inactive form in the cheese matrix of cheese manufactured in trial 3.

In conclusion, the results indicate that cells lysis indeed liberates the overproduced CBL-enzyme into the cheese matrix, but that this does not significantly enhance/alter flavour development compared to the reference cheese of trial 2. It is therefore surprisingly found that enhancement of lysis of CBL overproducing strains is not in all cases advantageous or necessary for the envisaged taste and flavour development in the cheese, and that the CBL activity should preferably be present in the cells at the moment of adding the said cells to the cheese milk, during the course of curd formation from the cheese milk and during ripening of the cheese product (steps 1-3 in figure 1).

## Claims

1. Method for the preparation of a cheese product, comprising the step of adding bacteria having cystathionine-β-lyase (CBL) activity to material from which the cheese product is formed, wherein the bacteria, when added to cheese milk in an amount of up to 1 X 10⁷ bacteria per ml cheese milk, have a specific CBL activity of at least 40 nmol mercaptide produced per ml cheese milk per minute.

2. Method according to claim 1, wherein the specific CBL activity is at least 100 nmol, preferably at least 200 nmol, more preferably at least 500 nmol, more preferably at least 1000 nmol, most preferably at least 1800 nmol mercaptide produced per ml cheese milk per minute.

3. Method according to claim 1 or 2, wherein per ml cheese milk, at least 10⁶, preferably between 2.0 - 4.0 x 10⁶ of the bacteria are added to the material from which the cheese product is formed.

4. Method according to any of the preceding claims, wherein at least a portion of the bacteria is added to the cheese milk, preferably at least 10⁶, more preferably between 2.0 -4.0 x 10⁶ bacteria per ml cheese milk.

5. Method according to any of the preceding claims, wherein the bacteria comprise
- a first number of a first group of bacteria, having, when added to cheese milk in an amount of up to 1 x 10⁷ bacteria per ml cheese milk, a specific CBL activity of at most 20 nmol mercaptide produced per ml cheese milk per minute, and
- a second number of a second group of bacteria, having, when added to cheese milk in an amount of up to 3 x 10⁶ bacteria per ml cheese milk, a specific CBL activity of at least 60 nmol mercaptide produced per ml cheese milk per minute,
wherein the ratio of the first number to the second number is between 1.5 - 5.0 : 1, preferably 1.8 - 3.0 : 1, most preferably 1.9 - 2.5 : 1.

6. Method according to claim 5, wherein the second group of bacteria has, when added to cheese milk in an amount of up to 3 x 10⁶ bacteria per ml cheese milk, a specific CBL activity of at least 100 nmol, preferably at least 200 nmol, more preferably at least 500 nmol, more preferably at least 1000 nmol, most preferably at least 1800 nmol mercaptide produced per ml cheese milk per minute.

7. Method according to any of the claims 5 - 6, wherein per ml cheese milk, at least 10⁶, preferably between 2.0 - 4.0 x 10⁶ bacteria of the second group are added to the material from which the cheese product is formed, preferably to cheese milk.

8. Method according to any of the claims 5 - 7, wherein the second group of bacteria comprises at least one CBL overproducing recombinant bacterial strain.

9. Method according to claim 8, wherein the expression of CBL from the CBL overproducing recombinant bacterial strain is inducable, preferably by nisin.

10. Method according to any of the claims 5 - 9, wherein the second group of bacteria comprises lactic acid bacteria, preferably all the bacteria of the second group being lactic acid bacteria.

11. Method according to any of the claims 8 - 10, wherein the CBL overproducing recombinant bacterial strain is *Lactococcus lactis* NZ9000, comprising a plasmid, wherein a CBL encoding nucleotide sequence is operably linked to a high expression promoter, functional in the said bacteria, the plasmid preferably being chosen from the group, consisting of pNZ8140 (CBS 109210) and pNZ8143 (CBS 109211).

12. Plasmid, chosen from the group, consisting of pNZ8140 (CBS 109210) and pNZ8143 (CBS 109211).

13. Use of a plasmid according to claim 12 in the preparation of a cheese product.

14. Cheese product, obtainable by the method according to any of the claims 1 - 12, having a specific CBL activity of at least 20 µmol, preferably at least 60 µmol, most preferably at least 80 µmol mercaptide produced per g cheese product per minute.

15. Cheese milk, having a specific CBL activity of at least 100 nmol, preferably at least 200 nmol, more preferably at least 500 nmol, more preferably at least 1000 nmol, most preferably at least 1800 nmol mercaptide produced per ml cheese milk per minute.
